(19) Europäisches Patentamt
European Patent Office
Office européen des brevets

(11) **EP 3 336 567 B1**

(12) **EUROPÄISCHE PATENTSCHRIFT**

(45) Veröffentlichungstag und Bekanntmachung des Hinweises auf die Patenterteilung:
**04.11.2020  Patentblatt 2020/45**

(51) Int Cl.:
*G01R 33/24* (2006.01)     *G01R 33/565* (2006.01)

(21) Anmeldenummer: **17182768.6**

(22) Anmeldetag: **24.07.2017**

(54) **BESTIMMUNG VON PHASENVERTEILUNGEN IN MR-BILDGEBUNGSVERFAHREN**

DETERMINATION OF PHASE DISTRIBUTIONS IN MR IMAGING METHOD

DÉTERMINATION DES RÉPARTITIONS DE PHASES DANS UN PROCÉDÉ D'IMAGERIE RM

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**20.06.2018  Patentblatt 2018/25**

(73) Patentinhaber: **Siemens Healthcare GmbH 91052 Erlangen (DE)**

(72) Erfinder:
• **Feiweier, Thorsten 91099 Poxdorf (DE)**
• **Niederlöhner, Daniel 91054 Erlangen (DE)**

(56) Entgegenhaltungen:
**EP-A1- 2 610 632     EP-A2- 0 390 175**

• **Grant Kaijuin Yang: "Computing Magnetic Susceptibility Maps from Gradient Recalled Echo MRI for use in Multiple Sclerosis Studies", Thesis, 28. März 2013 (2013-03-28), XP055456552, The Ohio State university, Columbus, Ohio, USA Gefunden im Internet: URL:http://citeseerx.ist.psu.edu/viewdoc/download?doi=10.1.1.836.8263&rep=rep1&type=pdf [gefunden am 2018-03-06]**
• **BORRELLO J A ET AL: "CHEMICAL SHIFT-BASED TRUE WATER AND FAT IMAGES: REGIONAL PHASE CORRECTION OF MODIFIED SPIN-ECHO MR IMAGES", RADIOLOGY, RADIOLOGICAL SOCIETY OF NORTH AMERICA, INC, US, Bd. 164, Nr. 2, 1. August 1987 (1987-08-01), Seiten 531-537, XP000995335, ISSN: 0033-8419**

**Beschreibung**

Die vorliegende Erfindung betrifft eine Bestimmung von Phasenverteilungen in MR-Bildgebungsverfahren, und insbesondere ein Verfahren zur Bestimmung einer Phasenverteilung eines Untersuchungsbereichs, welche zum Erzeugen eines MR-Bildes des Untersuchungsbereiches mittels einer MR-Anlage bestimmt wird.

Eine Reihe relevanter MR-Bildgebungsverfahren verwenden - teils zusätzlich zur Amplitude - die Phase des MR-Signals als grundlegende Information. Zu den relevanten MR-Bildgebungsverfahren zählen beispielsweise das B0-Mapping, welches eine räumliche Darstellung einer Variation der B0-Feldamplitude umfasst, die Suszeptibilitätsgewichtete Bildgebung (SWI), welche auf einer Bildwichtung unter Berücksichtigung von durch das Gewebe induzierten Variationen der lokalen B0-Feldamplitude (beispielsweise durch Präsenz von Hämoglobin im Falle von Blutungen) basiert, das Quantitative Suszeptibilitäts-Mapping (QSM), welches eine quantitative räumliche Ermittlung und Darstellung von lokalen, dipolartigen B0-Feldverzerrungen (beispielsweise durch Präsenz von Eisen, Calcium oder geeigneten Kontrastmitteln) beinhaltet, das Dixon-Verfahren, welches eine separate Darstellung der räumlichen Verteilung von Signalanteilen von beispielsweise Fett-und Wasser-gebundenen Wasserstoffkernen ermöglicht, die Thermometrie, bei welcher eine räumliche Temperaturverteilung bestimmt wird, und die Elastographie zur Bestimmung einer räumlichen Verteilung mechanischer Gewebeparameter.

Ein wesentlicher Schritt bei diesen Abbildungsverfahren ist die Ermittlung der räumlichen Verteilung einer Phasendifferenz relativ zu einer zuvor bestimmten oder festgelegten Referenzphase, d.h. einer räumliche Phasendifferenzverteilung. Diese Phasendifferenzverteilung stellt dann die Grundlage weiterer Verarbeitungsschritte dar. Die Schwierigkeit derartiger Verfahren begründet sich im limitierten Wertebereich der aus Messgrößen ermittelten Phaseninformation. Der Wertebereich von Phasen bzw. Phasendifferenzen ist beschränkt auf das Intervall $[-\pi, +\pi]$.

Für die Bestimmung der "tatsächlichen", d.h. absoluten Phasendifferenz zu einem Referenzwert kommen spezielle Verfahrensschritte zum Einsatz, die in der Regel Annahmen über die räumliche Entwicklung der Signalphase machen. Beispielsweise kann eine Annahme sein, dass sich die Signalphase zwischen benachbarten Bildpunkten nur wenig ändert. Insbesondere am Rand des Bildaufnahmebereichs kommt es - bedingt durch die Konstruktion des Grundfeldmagneten - unvermeidbar zu starken räumlichen Variationen der Signalphase. In diesen Bereichen kommt es somit häufig zu Fehlern bei der Bestimmung der tatsächlichen Phasendifferenz und in Folge zu fehlerhaften bzw. unpräzisen Darstellungen.

Im Stand der Technik werden beispielsweise "Phase Unwrapping"-Techniken verwendet, um - ausgehend von einer Referenzphase - die tatsächliche Phasendifferenz zu ermitteln. Dabei wird in der Regel eine langsame räumliche Variation der Phase angenommen. Sofern die Phasendifferenz eines benachbarten Bildpunktes einen definierten Schwellenwert, beispielsweise $+\pi$ oder $-\pi$ überscheitet, wird diesem Nachbarpunkt eine Zusatzphase, beispielsweise $+2\pi$ oder $-2\pi$ zugeordnet. Durch sukzessive Betrachtung weiterer Nachbarpunkte wird so schrittweise eine Phasenkarte, d.h. Phasenverteilung, mit einem nicht länger auf das Intervall das Intervall $[-\pi, +\pi]$ beschränkten Wertebereich aufgebaut.

Im Randbereich des Magneten kann allerdings der durch Inhomogenitäten des Magnetfeldes induzierte Anteil der Singalphase sehr schnell variieren, so dass fehlerhafte Zusatzphasen bestimmt werden. Durch die sukzessive Betrachtungsweise pflanzen sich diese Fehler über große Bildbereiche fort.

Aus der Druckschrift "Computing Magnetic Susceptibility Maps from Gradient Recalled Echo MRI for use in Multiple Sclerosis Studies" von Grant Yang, Thesis, The Ohio State University, 2013, ist ein Verfahren zum Bestimmen einer Phasenverteilung bekannt, wobei mittels MRT-Scans mit niedriger Auflösung und extrem kurzer Echozeit TE zusätzliche Daten zur Abschätzung einer B0-Feldkarte bereitgestellt werden, welche zum Entfernen von Phaseninhomogenitäten durch Magnetfeldinhomogenitäten aus dem MRT-Bild herangezogen werden.

Aus der Druckschrift "Chemical shift-based true water and fat images: regional phase correction of modified spin-echo MR images" von Joseph Borello et al. in Radiology 164.2 (1987): 531-537 ist ein Verfahren zum Korrigieren von B0-Magnetfeldinhomogenitäten in der MRT-Bildgebung mittels einer Dixon-Pulssequenz bekannt, wobei eine Phasenkorrektur mit Daten aus einer Phantommessung durchgeführt wird, welche Effekte von statischen Magnetfeldinhomogenitäten kompensiert.

Aus der Druckschrift EP 0 390 175 A2 ist ein Multi-Echo-NMR-Bildgebungsverfahren zum Erhalten von Bildern, die in Bezug auf zwei Arten von chemischen Verschiebungen voneinander getrennt sind, bekannt, wobei ein Bild eines homogenen Phantoms, welches durch die gleiche Messsequenz wie bei der Messung des zu untersuchenden Objekts gewonnen wird, zum Eliminieren von Fehlern, welche durch eine statische Magnetfeldverteilung verursacht werden, verwendet wird.

Als weiterer Ansatz sind Filtertechniken bekannt, wobei mit einem räumlichen Hochpass-Filter langsame räumliche Variationen der Phase unterdrückt werden, und so die hochfrequenten Anteile als "Nutzsignal" extrahiert werden. Im Randbereich des Magneten variiert allerdings auch der durch Inhomogenitäten des Magnetfeldes induzierte Anteil der Singalphase sehr schnell und wird so fehlerhaft dem Nutzsignal zugerechnet.

Daher besteht Bedarf nach einem verbesserten Verfahren zur Bestimmung von Phasenverteilungen in auf Phaseninformationen basierenden MR-Bildgebungsverfahren, welches die Robustheit und Präzision der MR-Verfahren

im Bereich schneller räumlicher Phasenvariationen, insbesondere am Rand von Bildaufnahmebereichen verbessert.

Diese Aufgabe wird mit den Merkmalen der unabhängigen Ansprüche gelöst. In den abhängigen Ansprüchen sind weitere Ausführungsformen der Erfindung beschrieben.

Gemäß einem ersten Aspekt der Erfindung wird ein Verfahren zur Bestimmung einer Phasenverteilung eines Untersuchungsbereichs mittels einer MR-Anlage bereitgestellt. Dabei wird eine Phasenverteilung innerhalb des Untersuchungsbereichs zum Erzeugen eines MR-Bildes bestimmt, wobei eine bekannte Magnetfeldverteilung in dem Untersuchungsbereich berücksichtigt wird, wobei die bekannte Magnetfeldverteilung in dem Untersuchungsbereich a priori in Absolutwerten bekannt ist und im Wesentlichen nicht durch ein Untersuchungsobjekt induziert ist. In einem ersten Schritt wird eine innerhalb des Untersuchungsbereichs gemessene Phasenverteilung bereitgestellt, welche zumindest auf der bekannten Magnetfeldverteilung im Untersuchungsbereich basiert.

In einem weiteren Schritt wird mindestens ein zweiter Phasenwert bereitgestellt. In einem zusätzlichen Schritt wird eine Phasendifferenzverteilung zwischen der ersten gemessenen Phasenverteilung und dem mindestens einen zweiten Phasenwert zum Erzeugen einer Kombinationsphasenverteilung bestimmt.

Dabei sind die Phasenwerte der Kombinationsphasenverteilung auf das Darstellungsintervall $[-\pi;+\pi]$ beschränkt. In einem zusätzlichen Schritt wird eine Korrekturphasenverteilung aus der bekannten Magnetfeldverteilung in dem Untersuchungsbereich berechnet, wobei die Phasenwerte der Korrekturphasenverteilung nicht auf das Darstellungsintervall $[-\pi; +\pi]$ beschränkt sind. In einem weiteren Schritt wird eine korrigierte Kombinationsphasenverteilung unter Verwenden der Korrekturphasenverteilung und der Kombinationsphasenverteilung erzeugt. Insbesondere kann dabei die die Korrekturphasenverteilung von der Kombinationsphasenverteilung subtrahiert werden, in anderen Worten separiert oder abgezogen werden, um eine korrigierte Kombinationsphasenverteilung zu erzeugen. Beispielsweise kann auch eine konjugiert-komplexe Multiplikation oder ein anderes mathematisches Verfahren zum Berücksichtigen der Korrekturphasenverteilung und der Kombinationsphasenverteilung durchgeführt werden. Dabei sind die Phasenwerte der korrigierten Kombinationsphasenverteilung auf das Darstellungsintervall $[-\pi;+\pi]$ beschränkt. In noch einem weiteren Schritt wird eine absolute Kombinationsphasenverteilung aus der korrigierten Kombinationsphasenverteilung durch eine Phase-Unwrapping-Technik erzeugt, wobei die Phasenwerte der absoluten Kombinationsphasenverteilung nicht auf das Darstellungsintervall $[-\pi;+\pi]$ beschränkt sind.

Das erfindungsgemäße Verfahren nutzt eine Kenntnis über die räumliche Verteilung eines Grundmagnetfeldes, insbesondere über räumlich schnell variierende Beiträge einer bekannte Magnetfeldverteilung in einem Randbereich eines Untersuchungsbereichs, und reduziert so die Fehler bei der Bestimmung von Phasenverteilungen in dem Randbereich. Insbesondere reduziert das erfindungsgemäße Verfahren die räumliche Variation einer Signalphase von gemessenen MR-Daten durch Separation eines Anteils von a priori bekannten räumlichen Variationen der Signalphase. Auf diese Weise wird eine gutartigere räumliche Phasenverteilung, die im Wesentlichen durch ein Objekt induziert ist erzeugt, mit welcher eine weniger fehleranfällige Bestimmung relativer Phasenbeziehungen in auf Phaseninformationen basierenden MR-Bildgebungsverfahren möglich ist. Im Ergebnis wird somit die Robustheit und Präzision auf Phaseninformationen basierenden MR-Bildgebungsverfahren insbesondere im Rand von Bildaufnahmebereichen verbessert.

Das Verfahren kann zusätzlich ein Erzeugen einer korrigierten absoluten Kombinationsphasenverteilung unter Verwenden der Korrekturphasenverteilung und der absoluten Kombinationsphasenverteilung umfassen. Insbesondere kann das Verfahren dabei ein Addieren der Korrekturphasenverteilung zu der absoluten Kombinationsphasenverteilung umfassen.

Die Korrekturphasenverteilung $\varphi_{korr}$ kann gemäß der Formel

$$\varphi_{korr}(\boldsymbol{r}) = \gamma \Delta B_0(\boldsymbol{r}) T_{eff}$$

bestimmt werden, wobei $\gamma$ das gyromagnetische Verhältnis, $\Delta B_0(\boldsymbol{r})$ eine Magnetfeldverteilung in dem Untersuchungsbereich, $\boldsymbol{r}$ der Ortsvektor im Untersuchungsbereich, und $T_{eff}$ eine effektive Evolutionszeit ist. Durch die oben genannte Formel kann die Korrekturphasenverteilung basierend auf der Magnetfeldverteilung und der effektiven Evolutionszeit einfach und schnell bestimmt werden.

Das Erzeugen der absoluten Kombinationsphasenverteilung kann basierend auf einer Phase-Unwrapping-Technik durchgeführt werden. Durch eine im Stand der Technik bekannte Phase-Unwrapping-Technik wird effizient eine absolute Phasenverteilung mit Phasenwerten, welche nicht auf ein festgelegtes Darstellungsintervall beschränkt sind, erzeugt.

Das Kombinieren der ersten gemessenen Phasenverteilung und des mindestens einen zweiten Phasenwertes kann ein Bestimmen einer Phasendifferenzverteilung sein. Die Phasendifferenzverteilung ist eine räumliche Zuordnung von Phasendifferenzen einzelner Voxel des Untersuchungsbereiches. Dadurch können Phasendifferenzen in MR-Bildgebungsverfahren zur Erzeugung von MR-Bildern schnell und effizient bestimmt werden.

Das Bestimmen der ersten gemessenen Phasenverteilung kann ein Messen einer Phasenverteilung des Untersuchungsbereichs, d.h. einer räumliche Verteilung von Phasenwerten, oder in anderen Worten einer räumlichen Zuordnung von Phasenwerten zu einzelnen Punkten in dem Untersuchungsbereich, nach einer ersten Evolutionszeitdauer umfassen. Dabei basieren die Phasenwerte der Phasenverteilung auf der bekannten Magnetfeldverteilung im Untersuchungsbereich. Das Bestimmen einer Phasenverteilung basierend auf einer Magnetfeldverteilung im Untersuchungsbereich nach einer ersten Evolutionszeitdauer wird in vielen MR-Bildgebungsverfahren zur Erzeugung von MR-Bildern benötigt und eine Phasenkombination kann somit schnell und effizient durchgeführt werden.

Das Bereitstellen mindestens eines zweiten Phasenwertes kann ein Bereitstellen einer zweiten gemessenen Phasenverteilung des Untersuchungsbereichs sein. Das Bereitstellen der zweiten gemessenen Phasenverteilung kann weiter ein Messen einer Phasenverteilung eines MR Bildes nach einer von der ersten Evolutionszeitdauer unterschiedlichen zweiten Evolutionszeitdauer sein. Das Bereitstellen mindestens eines zweiten Phasenwertes kann ein Festlegen eines globalen Phasenwertes des Untersuchungsbereiches sein. Eine Phasenverteilung kann damit in vielen MR-Bildgebungsverfahren zur Erzeugung von MR-Bildern schnell und effizient bestimmt werden.

Gemäß einem weiteren Aspekt wird eine MR-Anlage zur Bestimmung einer Phasenverteilung eines Untersuchungsbereichs bereitgestellt. Die MR-Anlage umfasst eine MR-Steuereinheit und eine Speichereinheit, wobei die Speichereinheit von der MR-Steuereinheit ausführbare Steuerinformationen speichert. Die MR-Anlage ist ausgebildet, bei der Ausführung der Steuerinformationen in der MR-Steuereinheit die Schritte des Verfahrens entsprechend den unter dem ersten Aspekt der Erfindung beschriebenen Merkmale auszuführen.

Für eine derartige MR-Anlage zur Bestimmung einer Phasenverteilung eines Untersuchungsbereichs können technische Effekte erzielt werden, die vergleichbar sind mit den technischen Effekten, die für das Verfahren gemäß dem ersten Aspekt obenstehend beschrieben wurden.

Gemäß einem weiteren Aspekt der Erfindung wird ein Computerprogrammprodukt bereitgestellt, welches ein Programm umfasst, das direkt in einen Speicher einer MR-Steuereinheit einer MR-Anlage ladbar ist, mit Programm-Mitteln, um die Schritte des Verfahrens entsprechend den unter dem ersten Aspekt der Erfindung beschriebenen Merkmale auszuführen, wenn das Programm in der MR-Steuereinheit der MR-Anlage ausgeführt wird.

Gemäß einem weiteren Aspekt der Erfindung wird ein elektronisch lesbarer Datenträger mit darauf gespeicherten elektronisch lesbaren Steuerinformationen bereitgestellt, welche derart ausgestaltet sind, dass sie bei Verwendung des Datenträgers in einer MR-Steuereinheit einer MR-Anlage das Verfahren entsprechend den unter dem ersten Aspekt der Erfindung beschriebenen Merkmalen durchführen.

Die oben dargelegten Merkmale und Merkmale, die nachfolgend beschrieben werden, können nicht nur in den entsprechenden explizit dargelegten Kombinationen verwendet werden, sondern auch in weiteren Kombinationen oder isoliert, ohne den Schutzumfang der vorliegenden Erfindung zu verlassen.

Kurze Beschreibung der Figuren

Die Erfindung wird nachfolgend unter Bezugnahme auf die beiliegenden Zeichnungen näher erläutert.

Figur 1 zeigt schematisch eine MR-Anlage, mit der ein Verfahren zur Bestimmung einer Phasenverteilung eines Untersuchungsbereichs (21) erfindungsgemäß durchgeführt werden kann.

Figur 2a zeigt eine zweidimensionale Darstellung einer Magnetfeldverteilung $B_M$ in einem Untersuchungsbereich, welche auf systemspezifischen Gegebenheiten der MR-Anlage basiert.

Figur 2b zeigt ein Liniendiagramm der in Figur 2a gezeigten Magnetfeldverteilung entlang einer zentralen Zeile der zweidimensionalen Darstellung der Figur 2a.

Figur 3a zeigt eine zweidimensionale Darstellung einer von einem Objekt induzierten Magnetfeldverteilung $\boldsymbol{B_P}$ in dem Untersuchungsbereich.

Figur 3b zeigt ein Liniendiagramm der in Figur 3a gezeigten Magnetfeldverteilung entlang einer zentralen Zeile der zweidimensionalen Darstellung der Figur 3a.

Figur 4a zeigt eine zweidimensionale Darstellung einer Überlagerung der Magnetfeldverteilungen $\boldsymbol{B_0 = B_M + B_P}$ der Figuren 2a und 3a.

Figur 4b zeigt ein Liniendiagramm der in Figur 4a gezeigten Magnetfeldverteilung entlang einer zentralen Zeile der zweidimensionalen Darstellung der Figur 4a.

Figur 5a zeigt eine zweidimensionale Darstellung einer Phasendifferenzverteilung $\Delta\varphi\boldsymbol{(r)}$ des Untersuchungsbe-

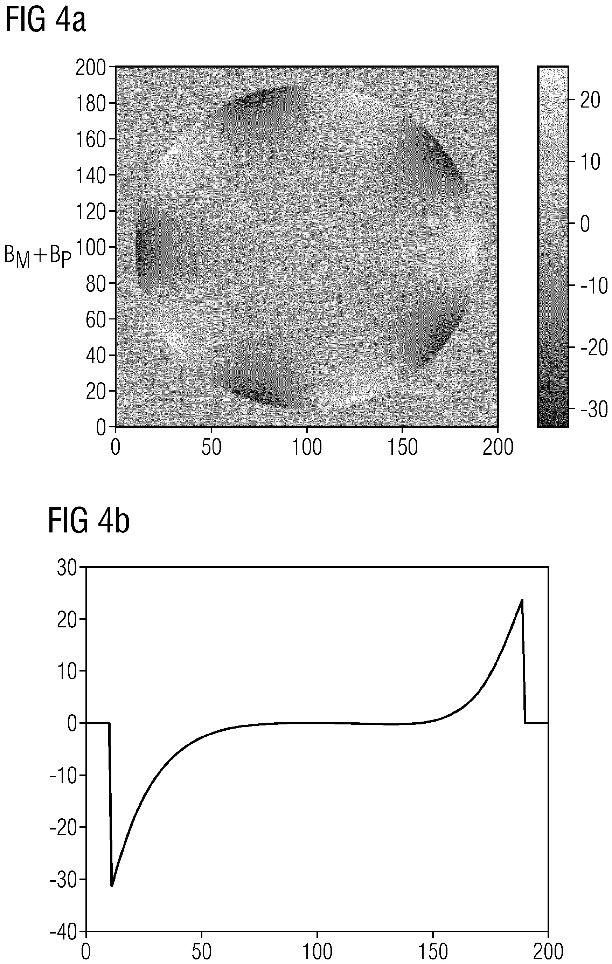

reichs, welche auf der in Figuren 4a und 4b gezeigten Magnetfeldverteilung basiert, und deren Phasenwerte auf das Intervall $[-\pi, +\pi]$ beschränkt sind.

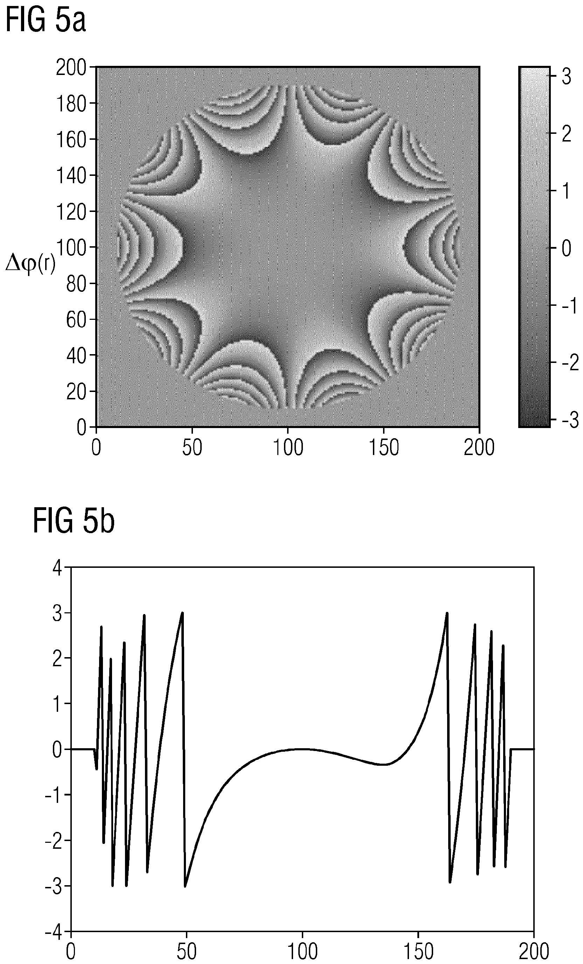

Figur 5b zeigt ein Liniendiagramm der in Figur 5a gezeigten Phasendifferenzverteilung $\Delta\varphi(\boldsymbol{r})$ entlang einer zentralen Zeile der zweidimensionalen Darstellung der Figur 5a.

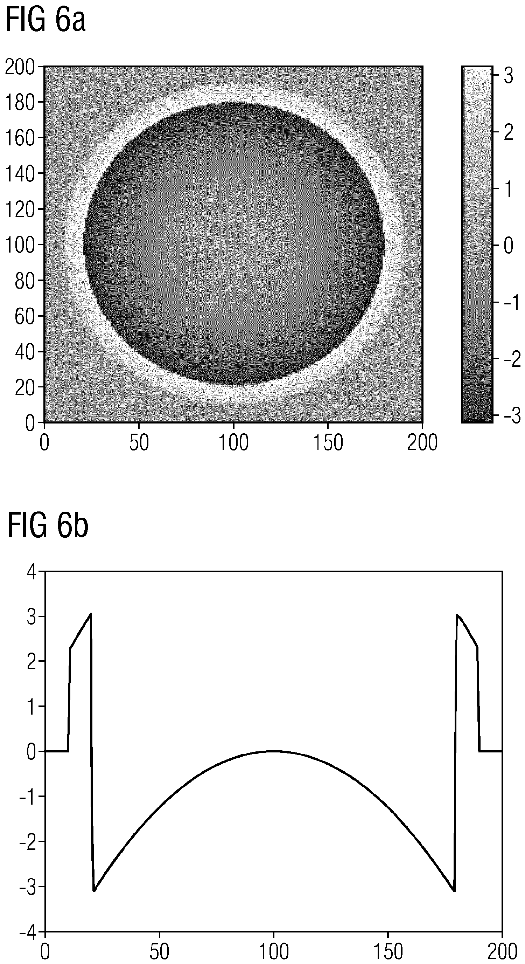

Figur 6a zeigt eine zweidimensionale Darstellung einer korrigierten Phasendifferenzverteilung des Untersuchungsbereichs, welche auf der in Figur 5a gezeigten Phasendifferenzverteilung basiert, und deren Phasenwerte auf das Intervall $[-\pi, +\pi]$ beschränkt sind.

Figur 6b zeigt ein Liniendiagramm der in Figur 6a gezeigten korrigierten Phasendifferenzverteilung des Untersuchungsbereichs entlang einer zentralen Zeile der zweidimensionalen Darstellung der Figur 6a.

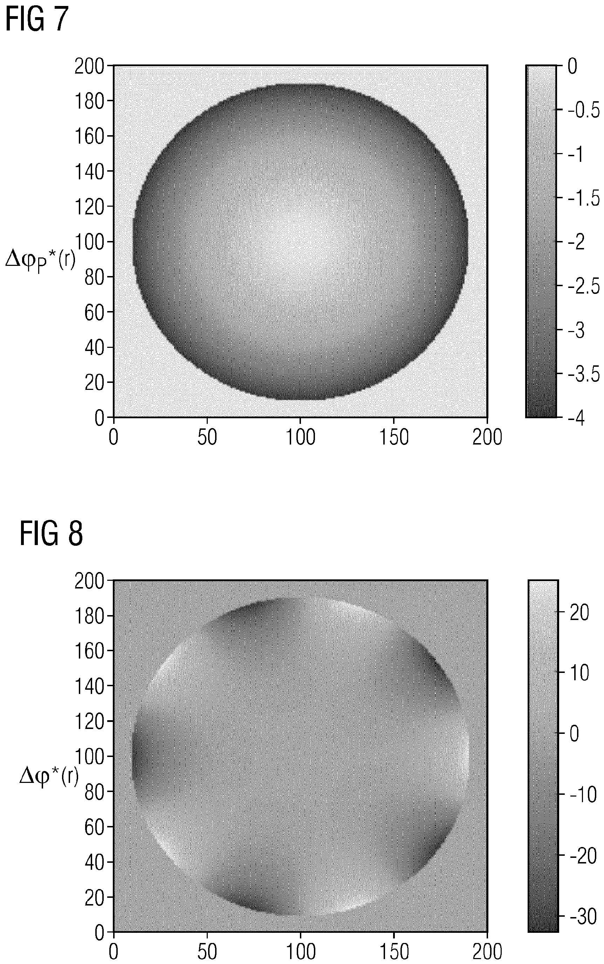

Figur 7 zeigt eine zweidimensionale Darstellung einer absoluten Phasendifferenzverteilung $\Delta\varphi^*(\boldsymbol{r})$ des Untersuchungsbereichs, welche durch eine Phase-Unwrapping-Technik aus der korrigierten Phasendifferenzverteilung der Figur 6 erzeugt wurde, und deren Phasenwerte nicht auf das Intervall $[-\pi, +\pi]$ beschränkt sind.

Figur 8 zeigt eine zweidimensionale Darstellung einer räumlichen Magnetfeldverteilung des $B_0$-Feldes, welche aus der absoluten Phasendifferenzverteilung der Figur 7 berechnet wurde.

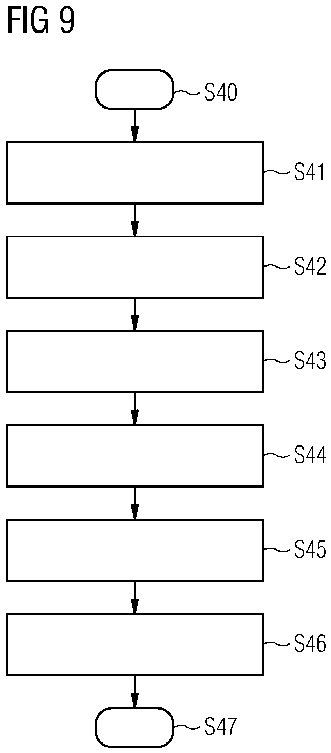

Figur 9 zeigt ein Flussdiagramm mit Schritten zur Durchführung eines Verfahrens zur Bestimmung einer Phasenverteilung eines Untersuchungsbereichs gemäß einem Ausführungsbeispiel der Erfindung.

Detaillierte Beschreibung

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

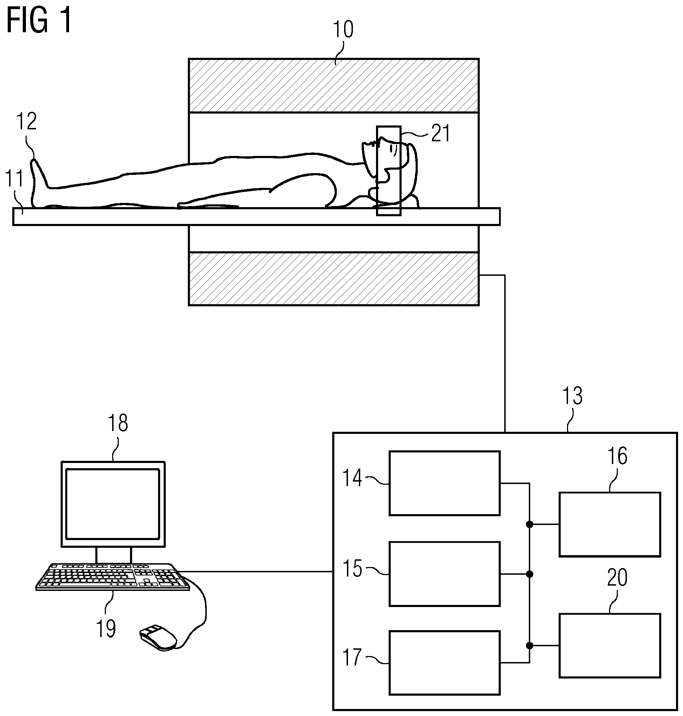

Figur 1 zeigt schematisch eine MR-Anlage, mit der ein Verfahren zur Bestimmung einer Phasenverteilung eines Untersuchungsbereichs 21 erfindungsgemäß durchgeführt werden kann.
Eine Untersuchungsperson 12, oder allgemeiner ein Untersuchungsobjekt, ist in den Tunnel der Anlage gefahren, wobei ein Untersuchungsbereich 21 durch die MR-Anlage untersucht wird. Die Magnetresonanzanlage weist einen Magneten 10 zur Erzeugung eines Grundfeldes B0 auf, wobei eine auf einer Liege 11 angeordnete Untersuchungsperson 12 in das Zentrum des Magneten gefahren wird, um dort ortscodierte Magnetresonanzsignale aus einem Untersuchungsabschnitt aufzunehmen. Durch Einstrahlen von Hochfrequenzpulsfolgen und Schalten von Magnetfeldgradienten kann die durch das Grundfeld B0 erzeugte Magnetisierung gestört werden durch Auslenkung der Kernspins aus der Gleichgewichtslage, und die bei der Rückkehr in die Gleichgewichtslage in Empfangsspulen induzierten Ströme können in Magnetresonanzsignale umgewandelt werden. Die allgemeine Funktionsweise zur Erstellung von MR-Bildern und die Detektion der Magnetresonanzsignale sind dem Fachmann bekannt, sodass auf eine detaillierte Erläuterung hiervon verzichtet wird.
Die Magnetresonanzanlage weist weiterhin eine MR-Steuereinheit 13 auf, die zur Steuerung des MR-Geräts verwendet wird. Die zentrale MR-Steuereinheit 13, welche derart ausgebildet ist, dass sie das unten beschriebene Verfahren zur Bestimmung einer Phasenverteilung eines Untersuchungsbereichs 21 durchführt, weist eine Gradientensteuerung 14 zur Steuerung und Schaltung der Magnetfeldgradienten auf und eine HF-Steuerung 15 zur Steuerung und Einstrahlung der HF-Pulse zur Auslenkung der Kernspins aus der Gleichgewichtlage. In einer Speichereinheit 16 können beispielsweise die für die Aufnahme der MR-Bilder notwendigen Bildgebungssequenzen abgespeichert werden, sowie alle Programme, die zum Betrieb der MR-Anlage notwendig sind. Eine Aufnahmeeinheit 17 steuert die Bildaufnahme und steuert damit in Abhängigkeit von den gewählten Bildgebungssequenzen die Abfolge der Magnetfeldgradienten und HF-Pulse und die Empfangsintervalle von MR-Signalen. Somit steuert die Aufnahmeeinheit 17 auch die Gradientensteuerung 14 und die HF-Steuerung 15. In einer Recheneinheit 20 können MR-Bilder berechnet werden, die auf eine Anzeige 18 angezeigt werden können, wobei eine Bedienperson über eine Eingabeeinheit 19 die MR-Anlage bedienen kann. Die Speichereinheit 16 kann Bildgebungssequenzen und Programmmodule aufweisen, die bei Ausführung in der Recheneinheit 20 von einem der gezeigten Module, das erfindungsgemäße Verfahren durchführen. Insbesondere speichert die Speichereinheit 16 dazu von der MR-Steuereinheit 13 ausführbare Steuerinformationen. Weiter ist die Aufnahmeeinheit 17 derart ausgebildet, dass sie das nachfolgend beschriebene Verfahren zur Bestimmung einer Phasenverteilung eines Untersuchungsbereichs 21 durchführen kann.
Erfindungsgemäß ist die MR-Anlage der Figur 1 derart ausgebildet, dass sie bei der Ausführung der Steuerinformationen in der MR-Steuereinheit 13 mindestens eine Phasenverteilung des Untersuchungsbereichs 21 zum Erzeugen eines MR-Bildes des Untersuchungsbereichs 21 bestimmt, und zum Erzeugen einer absoluten Phasenkombinationsverteilung, deren Phasenwerte nicht auf ein festgelegtes Darstellungsintervall eingeschränkt sind, eine bekannte Magnetfeldverteilung in dem Untersuchungsbereich 21 berücksichtigt.

Die Grundidee des erfindungsgemäßen Verfahrens ist die Separation von magnetinduzierten Phasendifferenzen, insbesondere von Phasendifferenzen, welche die durch stationäre Komponenten (z.B. HF-Ganzkörperspule, Verkleidung, Gradientenspule, ...) induziert werden, deren Magnetfeldverteilungen bekannt sind, sowie von Phasendifferenzen, die durch nicht-stationäre Komponenten (z.B. Patientenliege, HF-Lokalspulen, Lagerungshilfen) induziert werden, deren Lage und Magnetfeldverteilungen bekannt sind. Die Magnetinduzierten Phasendifferenz lässt sich ableiten aus einer bekannten räumlichen Magnetfeldverteilung $B_M(r)$, welche beispielsweise bei der Installation des MR-Systems, im Rahmen von regelmäßigen Service-Maßnahmen oder auch bereits vor der Auslieferung als "Design"- Parameter bestimmt wird. Die räumliche Verteilung der gewünschten Signal-Phasendifferenz $\Delta\varphi(r)=\varphi(r)-\varphi_{ref}$ ergibt sich aus dem komplexen MR-Signal mit Amplitude $A$ am Ort $r$ :

$$S(\boldsymbol{r}) = A(\boldsymbol{r}) \exp(i\,\varphi(\boldsymbol{r}))$$

Wobei weiterhin die folgende Relation gilt:

$$\varphi(\boldsymbol{r}) = \Delta\omega_0(\boldsymbol{r})T_{eff} + \varphi_{acq}(\boldsymbol{r}) + \varphi_{coil}(\boldsymbol{r}) = \gamma\Delta B_0(\boldsymbol{r})T_{eff} + \varphi_{acq}(\boldsymbol{r}) + \varphi_{coil}(\boldsymbol{r})$$

Hier bezeichnet $\Delta\omega_0(r)$ die Abweichung der lokalen Präzessionsfrequenz von einem Referenzwert (in der Regel die aktuelle Mittenfrequenz des HF-Empfangssystems), $\gamma$ das gyromagnetische Verhältnis und $T_{eff}$ eine effektive Evolutionszeit, die von der Messung abhängt. Für eine Gradientenecho-Messung gilt beispielsweise $T_{eff} = TE$, wobei die Echozeit $TE$ den zeitlichen Abstand zwischen HF-Anregungspuls und dem Zeitpunkt der Datenaufnahme definiert. Die lokale Feldabweichung $\Delta B_0(r)$ enthält an dieser Stelle die Magnetfeldverteilung $\Delta B_M(r)$ und durch ein Objekt induzierte Magnetfeldvariationen $\Delta B_P(r)$, aber auch weitere Einflussgrößen wie die effektive Änderung der Präzessionsfrequenz durch eine chemische Verschiebung $\Delta B_{CS}(r)$. Beispielsweise bei der MR-Elastographie kann dem Signal gezielt ein von $T_{eff}$ unabhängiger Phasenterm $\varphi_{acq}(r)$ aufgeprägt sein, der für den erwünschten Phasenkontrast sorgt. Durch Lokalspulen und HF-Transmissionseffekte kann ein zusätzlicher Phasenterm $\varphi_{coil}(r)$ auftreten, welcher sich beispielsweise durch Referenzmessungen bestimmen und berücksichtigen lässt.

Insbesondere am Rand des Bildgebungsbereichs weist die Signalphase $\Delta\varphi(r)$ aufgrund des Beitrags $\gamma\Delta B_M(r)T_{eff}$ schnelle räumliche Variationen auf. Das erfindungsgemäße Verfahren sieht nun vor, genau diesen bekannten Beitrag vor der Anwendung von "Phase-Unwrapping" Verfahren oder Filtertechniken zu separieren. Letztere arbeiten somit auf einer "gutartigeren" (räumlich weniger stark fluktuierenden)

$$\varphi_{eff}(\boldsymbol{r}) = \varphi(\boldsymbol{r}) - \gamma B_M(\boldsymbol{r})T_{eff}$$

Auf diese Weise lassen sich die gesuchten räumlichen Parameterverteilungen mit verbesserter Robustheit bzw. Präzision ermitteln. Falls notwendig - beispielsweise bei der Bestimmung von BO-Karten - kann der separierte Magnetanteil am Ende der Berechnung wieder zur Phasendifferenz addiert werden. Da das Magnetfeld als absolute Größe bekannt ist, kann dies ohne Beschränkung des Wertebereichs der Phase erfolgen.

Die Referenzphase $\varphi_{ref}$ kann einerseits willkürlich festgelegt werden oder durch die Phase an einer Position im Bild (oder einem Mittelwert über einen definierten Bereich) bestimmt sein. Andererseits kann sich die Referenzphase aber auch auf eine weitere Messung mit veränderten Einstellungen beziehen. Für die Ermittlung von BO-Karten können beispielsweise zwei Messungen mit unterschiedlichen effektiven Evolutionszeiten $T_{eff,1}$ und $T_{eff,2}$ durchgeführt werden. Die Signal-Phasendifferenz ergibt sich dann zu $\Delta\varphi(r) = \varphi_1(r) - \varphi_2(r)$, wobei folgende Relationen gelten:

$$S_1(\boldsymbol{r}) = A_1(\boldsymbol{r}) \exp\bigl(i\,\varphi_1(\boldsymbol{r})\bigr),$$

$$S_2(\boldsymbol{r}) = A_2(\boldsymbol{r}) \exp\bigl(i\,\varphi_2(\boldsymbol{r})\bigr),$$

$$\varphi_1(\boldsymbol{r}) = \Delta\omega_0(\boldsymbol{r})T_{eff,1} + \varphi_{acq,1}(\boldsymbol{r}) + \varphi_{coil,1}(\boldsymbol{r})$$
$$= \gamma\Delta B_0(\boldsymbol{r})T_{eff,1} + \varphi_{acq,1}(\boldsymbol{r}) + \varphi_{coil,1}(\boldsymbol{r}),$$

$$\varphi_2(\boldsymbol{r}) = \Delta\omega_0(\boldsymbol{r})T_{eff,2} + \varphi_{acq,2}(\boldsymbol{r}) + \varphi_{coil,2}(\boldsymbol{r})$$
$$= \gamma\Delta B_0(\boldsymbol{r})T_{eff,2} + \varphi_{acq,2}(\boldsymbol{r}) + \varphi_{coil,2}(\boldsymbol{r}).$$

Werden beide Messungen mit identischer Spulenkonfiguration $\varphi_{coil,1}(r)a = \varphi_{coil2}(r)$ und bis auf die Evolutionszeit identischem Ablauf $\varphi_{acq,1}(\mathbf{r}) = \varphi_{acq,2}(r)$ durchgeführt, ergibt sich die gesuchte Signal-Phasendifferenz auf der Formel

$$\Delta\varphi(\boldsymbol{r}) = \gamma\Delta B_0(\boldsymbol{r})(T_{eff,1} - T_{eff,2}).$$

Auch hier lässt sich der bekannte, räumlich schnell variierende Beitrag durch das Grundmagnetfeld durch die Formel

$$\Delta\varphi_{eff}(\boldsymbol{r}) = \gamma\Delta B_0(\boldsymbol{r})\big(T_{eff,1} - T_{eff,2}\big) - \gamma\Delta B_M(\boldsymbol{r})\big(T_{eff,1} - T_{eff,2}\big),$$

separieren, so dass man eine "gutartigere" räumliche Phasenverteilung für die weitere Datenverarbeitung erhält.
Zusammenfassend reduziert das erfindungsgemäße Verfahren die räumliche Variation einer Signalphase, und erlaubt so die robustere und präzisere Bestimmung relativer Phasenbeziehungen im Bild.
Exemplarisch soll das Verfahren für die Aufnahme absoluter B0-Karte erörtert werden. Ziel der Aufnahme von BO-Karten ist die Bestimmung von $\Delta B_0(r) = \Delta B_M(r) + \Delta B_P(r)$, wobei hier der Einfachheit halber auf die Betrachtung weiterer Einflussgrößen (z.B. $\Delta B_{SC}(r)$)) verzichtet wird. Dazu werden zumindest zwei Messungen mit unterschiedlichen effektiven Evolutionszeiten $T_{eff,1}$ und $T_{eff,2}$ aufgenommen und die räumliche Verteilung der relativen Signalphasen bestimmt durch die Formel

$$\Delta\varphi(\boldsymbol{r}) = \gamma\Delta B_0(\boldsymbol{r})\big(T_{eff,1} - T_{eff,2}\big) = \gamma\big(\Delta B_M(\boldsymbol{r}) + \Delta B_P(\boldsymbol{r})\big)\big(T_{eff,1} - T_{eff,2}\big).$$

Im Stand der Technik würde man die gewünschte B0-Information aus der folgenden Relation bestimmen:

$$\Delta B_0(\boldsymbol{r}) = \Delta\varphi(\boldsymbol{r}) \, / \, \big(\gamma\,\big(T_{eff,1} - T_{eff,2}\big)\big)$$

Da die Phasendifferenz nur einen Wertebereich von $-\pi$ und $+\pi$ hat, beschränkt sich der direkt zugängliche Wertebereich für die Feldkarte auf $+- \pi\, /\, (\gamma\,(T_{ett,1} - T_{eff,2}))$. Misst man beispielsweise mit $T_{eff,1}$ = 4.8 ms und $T_{eff,2}$ = 9.6 ms, ergibt sich ein Wertebereich von etwa +/- 2.4 µT. Bei einer Grundfeldstärke von 1.5 T entspricht dies einer relativen Feldänderung von +/- 1.6 ppm. Am Rand des Bildgebungsbereichs können die Inhomogenitäten des Grundfeldmagneten durchaus einige zehn ppm betragen. Im Ergebnis lässt sich diese Größenordnung mit der Messung also nicht mehr direkt abbilden, so dass eine eindeutige Bestimmung der B0-Amplitude nicht direkt möglich ist. Das folgende zweidimensionale Beispiel nimmt eine Magnetinduzierte Feldverteilung gemäß einer sphärisch-harmonischen Funktion der Art $\Delta B_M(x,y) = B_{M,0}\,{}^*\cos(5^*\alpha)\,{}^*\,(r/r_0)^5$ an, wobei $\alpha$ der Winkel in der Ebene, r der Abstand zur Mitte, $r_0$ ein Referenzradius und $B_{M,0}$ ein Skalierungsfaktor ist. Dem überlagert sei eine Objekt-induzierte Feldverteilung gemäß einer quadratischen Funktion $\Delta B_P(x,y) = B_{P,0}\,{}^*\,(r/r_0)^2$.
Die tatsächlichen physikalischen Felder haben die folgenden Formen, wobei die Einheiten zur Darstellung willkürlich gewählt und in allen Figuren einheitlich sind.

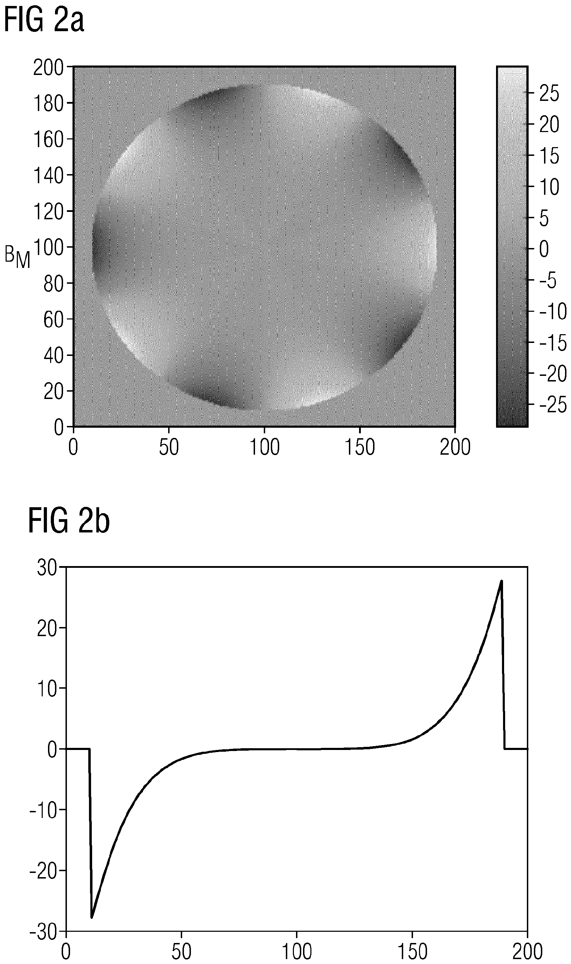

Figur 2a zeigt eine zweidimensionale Darstellung einer Magnetfeldverteilung $B_M$ in einem Untersuchungsbereich, welche auf systemspezifischen Gegebenheiten der MR-Anlage, wie zum Beispiel der Konstruktion des Grundfeldmagneten, basiert. Die gezeigte Magnetfeldverteilung $B_M$ weist in den Randbereichen der kreisförmigen Darstellung eine starke räumliche Variation mit hohen räumlicher Frequenz der Magnetfeldstärke auf.
Figur 2b zeigt ein Liniendiagramm der in Figur 2a gezeigten Magnetfeldverteilung entlang einer zentralen Zeile der zweidimensionalen Darstellung der Figur 2a.

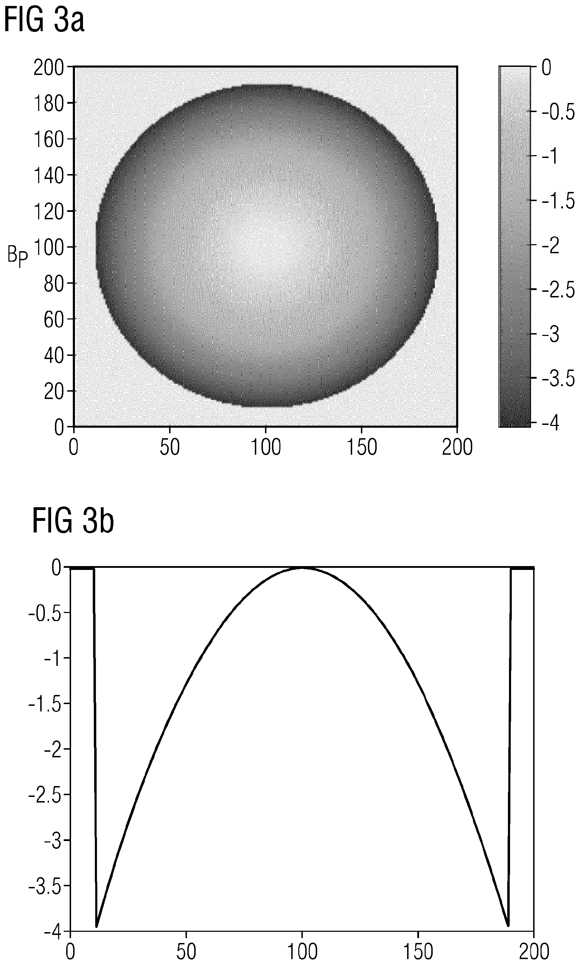

Figur 3a zeigt eine zweidimensionale Darstellung einer durch ein Objekt induzierte Magnetfeldverteilung $B_p$ in einem Untersuchungsbereich. Die gezeigte Magnetfeldverteilung $B_p$ weist in den Randbereichen der kreisförmigen Darstellung eine gleichmäßige Magnetfeldstärke auf.

Figur 3b zeigt ein Liniendiagramm der in Figur 3a gezeigten Magnetfeldverteilung entlang einer zentralen Zeile der zweidimensionalen Darstellung der Figur 3a.

Figur 4a zeigt eine zweidimensionale Darstellung einer Überlagerung der Magnetfeldverteilungen $B_0 = B_M + B_P$ der Figuren 2a und 3a. In der überlagerten Magnetfeldverteilung $B_0 = B_M + B_P$ überwiegt der Einfluss der Magnetfeldverteilung $B_M$, sodass die Randbereiche der kreisförmigen Darstellung ebenfalls eine starke räumliche Variation mit hohen räumlicher Frequenz der Magnetfeldstärke aufweisen.

Figur 4b zeigt ein Liniendiagramm der in Figur 4a gezeigten Magnetfeldverteilung entlang einer zentralen Zeile der zweidimensionalen Darstellung der Figur 4a.

Eine Messung einer Phasenverteilung durch die MR-Anlage kann nur die überlagerte Feldverteilung $\Delta B_0(x,y)$ über die Bestimmung einer Phasendifferenz $\Delta \varphi(\boldsymbol{r})$ erfassen, und liefert Daten mit einem eingeschränkten Wertebereich. Auswertungsroutinen "sehen" also die folgende räumliche Phasenverteilung, welche in Figur 5a dargestellt ist.

Figur 5a zeigt eine zweidimensionale Darstellung einer Phasendifferenzverteilung $\Delta \varphi(\boldsymbol{r})$ des Untersuchungsbereichs, welche auf der in Figuren 4a und 4b gezeigten Magnetfeldverteilung basiert, und deren Phasenwerte auf das Intervall $[-\pi, +\pi]$ beschränkt sind.

Figur 5b zeigt ein Liniendiagramm der in Figur 5a gezeigten Phasendifferenzverteilung $\Delta \varphi(\boldsymbol{r})$ entlang einer zentralen Zeile der zweidimensionalen Darstellung der Figur 5a.

In den Randbereichen der korrigierten Phasendifferenzverteilung der Figuren 5a und 5b ist eine Vielzahl von Phasensprüngen zu erkennen. Diese Phasensprünge werden durch die Beschränkung der Phasenwerte auf das Intervall $[-\pi, +\pi]$ verursacht.

Das Problem der uneindeutigen Werte im Randbereich ist klar zu erkennen. Im Stand der Technik müssen diese schnellen räumlichen Variationen durch geeignete Bildverarbeitungsverfahren (z.B. "Phase Unwrapping") erfasst und richtig zugeordnet werden, was - insbesondere bei rauschbehafteten Daten - zu Fehlern führen kann.

Erfindungsgemäß wird von der ermittelten Phasendifferenz $\Delta \varphi(\boldsymbol{r})$ zunächst der bekannte Beitrag $\Delta \varphi_M(\boldsymbol{r}) = \gamma B_M(\boldsymbol{r}) (T_{eff,1} - T_{eff,2})$, des Magneten separiert. Dabei wird die Magnetinduzierte Phase auf den Wertebereich zwischen $-\pi$ und $+\pi$ beschränkt. Auf diese Weise erhält man eine gutartigere räumliche Phasenverteilung, die im Wesentlichen durch ein Objekt induziert ist, wie in Figur 6 gezeigt.

Figur 6a zeigt eine zweidimensionale Darstellung einer korrigierten Phasendifferenzverteilung des Untersuchungsbereichs, welche auf der in Figur 5a gezeigten Phasendifferenzverteilung basiert, und deren Phasenwerte auf das Intervall $[-\pi, +\pi]$ beschränkt sind.

Figur 6b zeigt ein Liniendiagramm der in Figur 6a gezeigten korrigierten Phasendifferenzverteilung des Untersuchungsbereichs entlang einer zentralen Zeile der zweidimensionalen Darstellung der Figur 6a.

Es ist klar erkenntlich, dass das Problem uneindeutiger Werte im Randbereich deutlich weniger ausgeprägt ist. Dadurch gelingt die Zuordnung einer absoluten Phasendifferenz - beispielsweise durch "Phase Unwrapping" Verfahren -auch bei rauschbehafteten Daten mit hoher Wahrscheinlichkeit. Um aus der korrigierten Phasendifferenzverteilung der Figur 6 eine kontinuierliche Phasendifferenzverteilung, welche in dem Ausführungsbeispiel der absoluten Kombinationsphasenverteilung entspricht, ohne Begrenzung auf das Intervall $[-\pi, +\pi]$ zu erzeugen, wird in einem nächsten Schritt eine Phase-Unwrapping-Technik auf die korrigierte Phasendifferenzverteilung angewandt. Auch Filterverfahren können zum Erzeugen einer kontinuierlichen Phasendifferenzverteilung angewandt werden, wie im Stand der Technik bekannt. Als Resultat erhält man die Objekt-induzierte absolute Phasendifferenzverteilung $\Delta \varphi^*(\boldsymbol{r})$, welche in dem Ausführungsbeispiel der absoluten Kombinationsphasenverteilung entspricht, ohne Einschränkung des Wertebereichs, wie in Figur 7 gezeigt.

Figur 7 zeigt eine zweidimensionale Darstellung einer absoluten objekt-induzierten Phasendifferenzverteilung $\Delta \varphi^*(\boldsymbol{r})$, welche in dem Ausführungsbeispiel der absoluten Kombinationsphasenverteilung entspricht, des Untersuchungsbereichs, welche durch eine Phase-Unwrapping-Technik aus der korrigierten Phasendifferenzverteilung der Figur 6 erzeugt wurde, und deren Phasenwerte nicht auf das Intervall $[-\pi, +\pi]$ beschränkt sind.

Als letzter Schritt findet der zuvor separierte Magnetanteil wieder Berücksichtigung. Hierbei ist wesentlich, dass dieser Beitrag in seinem Wertebereich nicht eingeschränkt ist, da der Absolutwert von $B_M(r)$ a priori bekannt ist. Man erhält so unmittelbar die räumliche Verteilung der absoluten Phasendifferenzen und daraus abgeleitet die gesuchte räumliche B0-Verteilung, wie in Figur 8 gezeigt aus der Formel:

$$\Delta \varphi^*(\boldsymbol{r}) = \Delta \varphi_P^*(\boldsymbol{r}) + \gamma \Delta B_M(\boldsymbol{r}) \left( T_{eff,1} - T_{eff,2} \right)$$

Figur 8 zeigt eine zweidimensionale Darstellung einer räumlichen Magnetfeldverteilung des $B_0$-Feldes, welche aus der absoluten Phasendifferenzverteilung der Figur 7, welche in dem Ausführungsbeispiel der absoluten Kombinationsphasenverteilung entspricht, berechnet wurde.

Figur 9 zeigt ein Flussdiagramm mit Schritten zur Durchführung eines Verfahrens zur Bestimmung einer Phasenverteilung eines Untersuchungsbereichs gemäß einem Ausführungsbeispiel der Erfindung.

Das Verfahren beginnt in Schritt S40. In Schritt S41 wird eine erste gemessene Phasenverteilung des Untersuchungsbereichs bereitgestellt. In Schritt S42 wird mindestens ein zweiter Phasenwert bereitgestellt. In Schritt S43 wird die erste gemessene Phasenverteilung und der mindestens eine zweite Phasenwert miteinander kombiniert, zum Erzeugen einer Kombinationsphasenverteilung. Dabei sind die Phasenwerte der Kombinationsphasenverteilung auf ein festgelegtes Darstellungsintervall beschränkt. In Schritt S44 wird eine Korrekturphasenverteilung erzeugt, welche auf der bekannten Magnetfeldverteilung in dem Untersuchungsbereich basiert. Dabei sind die Phasenwerte der Korrekturphasenverteilung nicht auf das festgelegte Darstellungsintervall beschränkt. In Schritt S45 wird eine korrigierte Kombinationsphasenverteilung unter Verwenden der Korrekturphasenverteilung und der Kombinationsphasenverteilung erzeugt, wobei die Phasenwerte der korrigierten Kombinationsphasenverteilung auf das festgelegte Darstellungsintervall beschränkt sind. In Schritt S46 wird eine absolute Kombinationsphasenverteilung aus der korrigierten Kombinationsphasenverteilung erzeugt, wobei die Phasenwerte der absoluten Kombinationsphasenverteilung nicht auf das festgelegte Darstellungsintervall beschränkt sind. Das Verfahren endet in Schritt S47.

In einem Ausführungsbeispiel wird das erfindungsgemäße Verfahren bei Aufnahmen mit mehreren Spulenelementen zunächst auf die Phasendaten jedes einzelnen Elementes angewendet. Danach findet eine herkömmliche Kombination unter Berücksichtigung der Phaseninformation statt.

In einem anderen Ausführungsbeispiel werden zunächst die Daten aller Spulenelemente unter Berücksichtigung der Phaseninformation auf herkömmliche Weise kombiniert. Danach wird das erfindungsgemäße Verfahren auf die kombinierten Phasendaten angewendet.

In einem weiteren Ausführungsbeispiel werden mehr als zwei Messungen mit unterschiedlichen effektiven Evolutionszeiten durchgeführt, wobei das erfindungsgemäße Verfahren auf beliebige Kombinationen der Signalphasen angewendet wird.

In einem zusätzlichen Ausführungsbeispiel wird das erfindungsgemäße Verfahren bei Durchführung von mehr als einer Messung mit unterschiedlichen effektiven Evolutionszeiten sowohl auf die Einzelmessungen (d.h. vor der Kombination der Signalphasen) als auch auf die kombinierten Messungen angewandt.

Aufgrund der verbesserten Robustheit gegenüber den starken Magnetfeldvariationen im Randbereich ermöglicht das erfindungsgemäße Verfahren auch bei gröberen Voxeln innerhalb des Untersuchungsbereiches eine hohe Präzision der Resultate. Dies erlaubt beispielsweise die beschleunigte Aufnahme eines großen räumlichen Bereichs mit reduzierter Auflösung, beispielsweise für die Aufnahme von BO-Karten im Zuge von Justageverfahren.

Neben dem stationären Grundfeldmagneten können weitere Bauteile zu lokalen Variationen des B0-Feldes (und somit zur Phasenvariation) beitragen. Sofern der Einfluss dieser Bauteile auf die räumliche Magnetfeldverteilung und die Lage der Bauteile relativ zum Grundfeldmagneten bekannt sind, können auch diese Anteile bei der erfindungsgemäßen Separation berücksichtigt werden. Beispiele für stationäre Bauteile sind eine Verkleidung, eine HF-Ganzkörperspule, Gradientenspulen und Shim-Spulen. Bei Gradienten- und Shim-Spulen können die variablen, bekannten Einstellungen der von ihnen erzeugten Zusatzfelder mit linearer Geometrie oder auch höheren Ordnungen berücksichtigt werden. Beispiele für nicht-stationäre Bauteile mit bekannter Lage sind eine Patientenliege, Positionierungshilfen und lokale HF-Spulen.

Zusammenfassend wird ein Verfahren zur Bestimmung einer Phasenverteilung eines Untersuchungsbereichs durchgeführt. Eine Kombination von Phasenverteilungen des Untersuchungsbereichs, welche zum Erzeugen eines MR-Bildes des Untersuchungsbereichs mittels einer MR-Anlage bestimmt wurden, wird durchgeführt, wobei eine bekannte Magnetfeldverteilung in dem Untersuchungsbereich berücksichtigt wird. Erfindungsgemäß wird ein Anteil von a priori bekannten räumlichen Variationen der Signalphase von gemessenen Daten separiert, um eine gutartigere räumliche Phasenverteilung, die im Wesentlichen durch ein Objekt induziert ist, zu erzeugen.

Erfindungsgemäß nutzt das Verfahren Vorwissen über eine räumliche Verteilung eines Grundmagnetfeldes, insbesondere räumlich schnell variierende Beiträge in einem Randbereich eines Untersuchungsbereichs aufgrund von systembedingten bekannten Magnetfeldvariationen, wie zum Beispiel durch die Konstruktion des Grundfeldmagneten bedingte Magnetfeldvariationen, und reduziert so die Fehler bei der Bestimmung von Phasendifferenzen im Randbereich des Untersuchungsbereichs.

**Patentansprüche**

1. Verfahren zur Bestimmung einer Phasenverteilung eines Untersuchungsbereichs, welche zum Erzeugen eines MR-Bildes des Untersuchungsbereiches (21) mittels einer MR-Anlage bestimmt wird, wobei eine bekannte Magnetfeldverteilung in dem Untersuchungsbereich (21) berücksichtigt wird, umfassend die folgenden Schritte:

- Bereitstellen einer ersten gemessenen Phasenverteilung, welche zumindest auf der bekannten Magnetfeldverteilung im Untersuchungsbereich basiert (21, S41);
- Bereitstellen mindestens eines zweiten Phasenwertes des Untersuchungsbereiches (21, S42);
- Bestimmen einer Phasendifferenzverteilung zwischen der ersten gemessenen Phasenverteilung und dem mindestens einen zweiten Phasenwertes zum Erzeugen einer Kombinationsphasenverteilung, wobei die Phasenwerte der Kombinationsphasenverteilung auf das Darstellungsintervall [$-\pi$, $+\pi$] beschränkt sind (S43);
- Berechnen einer Korrekturphasenverteilung aus der bekannten Magnetfeldverteilung in dem Untersuchungsbereich (21), wobei die Phasenwerte der Korrekturphasenverteilung nicht auf das Darstellungsintervall [$-\pi$, $+\pi$] beschränkt sind(S44);
- Erzeugen einer korrigierten Kombinationsphasenverteilung unter Verwenden der Korrekturphasenverteilung und der Kombinationsphasenverteilung, wobei die Phasenwerte der korrigierten Kombinationsphasenverteilung auf das Darstellungsintervall [$-\pi$, $+\pi$] beschränkt sind (S45); und
- Erzeugen einer absoluten Kombinationsphasenverteilung aus der korrigierten Kombinationsphasenverteilung durch eine Phase-Unwrapping-Technik, wobei die Phasenwerte der absoluten Kombinationsphasenverteilung nicht auf das Darstellungsintervall [$-\pi$, $+\pi$] beschränkt sind (S46) ;

**dadurch gekennzeichnet, dass** die bekannte Magnetfeldverteilung in dem Untersuchungsbereich (21) a priori in Absolutwerten bekannt ist und im Wesentlichen nicht durch ein Untersuchungsobjekt induziert ist.

2. Verfahren nach Anspruch 1, wobei das Verfahren weiter den folgenden Schritt umfasst:

- Erzeugen einer korrigierten absoluten Kombinationsphasenverteilung, welche nicht auf das Darstellungsintervall [$-\pi$, $+\pi$] beschränkt ist, durch Hinzufügen der Korrekturphasenverteilung zu der absoluten Kombinationsphasenverteilung, zum Bestimmen einer B0-Feldverteilung.

3. Verfahren nach Anspruch 1 oder 2, wobei die Korrekturphasenverteilung $\varphi_{korr}(r)$ nach der Formel

$$\varphi_{korr}(\boldsymbol{r}) = \gamma \Delta B_0(\boldsymbol{r}) T_{eff}$$

bestimmt wird, wobei $\gamma$ das gyromagnetische Verhältnis, $\Delta B_0(r)$ eine Magnetfeldverteilung in dem Untersuchungsbereich (21), welche nicht durch ein Untersuchungsobjekt induziert ist, $r$ der Ortsvektor im Untersuchungsbereich (21), und $T_{eff}$ eine effektive Evolutionszeit ist.

4. Verfahren nach einem der vorhergehenden Ansprüche, wobei das Bestimmen der ersten gemessenen Phasenverteilung ein Messen einer Phasenverteilung eines MR-Bildes des Untersuchungsbereichs (21) nach einer ersten Evolutionszeitdauer ist.

5. Verfahren nach einem der Ansprüche 1 bis 4, wobei das Bereitstellen mindestens eines zweiten Phasenwertes ein Bereitstellen einer zweiten gemessenen Phasenverteilung des Untersuchungsbereichs (21) ist.

6. Verfahren nach Anspruch 5, wobei das Bereitstellen der zweiten gemessenen Phasenverteilung, ein Messen einer Phasenverteilung eines MR-Bildes nach einer von der ersten Evolutionszeitdauer unterschiedlichen zweiten Evolutionszeitdauer ist.

7. Verfahren nach einem der Ansprüche 1 bis 4, wobei das Bereitstellen mindestens eines zweiten Phasenwertes des Untersuchungsbereiches (21) ein Festlegen eines globalen Phasenwertes ist.

8. MR-Anlage zur Bestimmung einer Phasenverteilung eines Untersuchungsbereichs (21), wobei die MR-Anlage eine MR-Steuereinheit (13) und eine Speichereinheit (16) aufweist, wobei die Speichereinheit (16) von der MR-Steuereinheit (13) ausführbare Steuerinformationen speichert, und wobei die MR-Anlage ausgebildet ist, bei der Ausführung der Steuerinformationen in der MR-Steuereinheit (13) folgende Schritte auszuführen:

- Bereitstellen einer ersten gemessenen Phasenverteilung des Untersuchungsbereichs (21), welche zumindest auf der bekannten Magnetfeldverteilung im Untersuchungsbereich basiert;

- Bereitstellen mindestens eines zweiten Phasenwertes des Untersuchungsbereiches (21);
- Bestimmen einer Phasendifferenzverteilung zwischen der ersten gemessenen Phasenverteilung und dem mindestens einen zweiten gemessenen Phasenwertes zum Erzeugen einer Kombinationsphasenverteilung, wobei die Phasenwerte der Kombinationsphasenverteilung auf das Darstellungsintervall $[-\pi, +\pi]$ beschränkt sind;
- Berechnen einer Korrekturphasenverteilung aus einer in Absolutwerten a priori bekannten abweichenden Magnetfeldverteilung in dem Untersuchungsbereich (21), welche im Wesentlichen nicht durch ein Untersuchungsobjekt induziert ist, wobei die Phasenwerte der Korrekturphasenverteilung nicht auf das Darstellungsintervall $[-\pi, +\pi]$ beschränkt sind(S44);
- Erzeugen einer korrigierten Kombinationsphasenverteilung unter Verwenden der Korrekturphasenverteilung und der Kombinationsphasenverteilung, wobei die Phasenwerte der korrigierten Kombinationsphasenverteilung auf das Darstellungsintervall $[-\pi, +\pi]$ beschränkt sind; und
- Erzeugen einer absoluten Kombinationsphasenverteilung aus der korrigierten Kombinationsphasenverteilung durch eine Phase-Unwrapping-Technik, wobei die Phasenwerte der absoluten Kombinationsphasenverteilung nicht auf das Darstellungsintervall $[-\pi, +\pi]$ beschränkt sind;

**dadurch gekennzeichnet, dass** die bekannte Magnetfeldverteilung in dem Untersuchungsbereich (21) a priori in Absolutwerten bekannt ist und im Wesentlichen nicht durch ein Untersuchungsobjekt induziert ist.

9. MR-Anlage zur Bestimmung einer Phasenverteilung eines Untersuchungsbereichs (21), wobei die MR-Anlage eine MR-Steuereinheit (13) und eine Speichereinheit (16) aufweist, wobei die Speichereinheit (16) von der MR-Steuereinheit (13) ausführbare Steuerinformationen speichert, und wobei die MR-Anlage ausgebildet ist, bei der Ausführung der Steuerinformationen in der MR-Steuereinheit (13) das Verfahren eines der Ansprüche 2 bis 7 auszuführen.

10. Computerprogrammprodukt, welches ein Programm umfasst, das direkt in einen Speicher einer MR-Steuereinheit (13) einer MR-Anlage ladbar ist, mit Programm-Mitteln, um die Schritte des Verfahrens nach einem der Ansprüche 1 bis 7 auszuführen, wenn das Programm in der MR-Steuereinheit (13) der MR-Anlage ausgeführt wird.

11. Elektronisch lesbarer Datenträger mit darauf gespeicherten elektronisch lesbaren Steuerinformationen, welche derart ausgestaltet sind, dass sie bei Verwendung des Datenträgers in einer MR-Steuereinheit (13) einer MR-Anlage das Verfahren nach einem der Ansprüche 1 bis 7 durchführen.

## Claims

1. Method for the determination of a phase distribution of a region of interest, which is determined in order to generate an MR image of the region of interest (21) by means of an MR system, wherein a known magnetic field distribution in the region of interest (21) is taken into account, comprising the following steps:

   - provision of a first measured phase distribution which is based at least on the known magnetic field distribution in the region of interest (21, S41);
   - provision of at least one second phase value of the region of interest (21, S42);
   - determination of a phase-difference distribution between the first measured phase distribution and the at least one second phase value to generate a combination-phase distribution, wherein the phase values of the combination-phase distribution are restricted to the presentation interval [-n;+n] (S43);
   - calculation of a correction-phase distribution from the known magnetic field distribution in the region of interest (21), wherein the phase values of the correction-phase distribution are not restricted to the presentation interval $[-\pi;+\pi]$ (S44);
   - generation of a corrected combination-phase distribution using the correction-phase distribution and the combination-phase distribution, wherein the phase values of the corrected combination-phase distribution are restricted to the presentation interval $[-\pi;+\pi]$ (S45); and
   - generation of an absolute combination-phase distribution from the corrected combination-phase distribution using a phase-unwrapping technique, wherein the phase values of the absolute combination-phase distribution are not restricted to the presentation interval $[-\pi;+\pi]$ (S46);

   **characterised in that** the known magnetic field distribution in the region of interest (21) is known a priori in absolute values and is substantially not induced by an object of interest.

2. Method according to claim 1, wherein the method further comprises the following step:

   - generation of a corrected absolute combination-phase distribution which is not restricted to the presentation interval [-π;+π], by addition of the correction-phase distribution to the absolute combination-phase distribution to determine a B0 field distribution.

3. Method according to claim 1 or 2, wherein the correction-phase distribution ($\varphi_{korr}(r)$) is determined according to the formula

$$\varphi_{korr}(\boldsymbol{r}) = \gamma \Delta B_0(\boldsymbol{r}) T_{eff},$$

   wherein $\gamma$ is the gyromagnetic ratio, $\Delta B_0(\boldsymbol{r})$ is a magnetic field distribution in the region of interest (21) which is not induced by an object of interest, $\boldsymbol{r}$ is the position vector in the region of interest (21), and $T_{eff}$ is an effective evolution time.

4. Method according to one of the preceding claims, wherein the determination of the first measured phase distribution is a measurement of a phase distribution of an MR image of the region of interest (21) after a first evolution period.

5. Method according to one of claims 1 to 4, wherein the provision of at least one second phase value is a provision of a second measured phase distribution of the region of interest (21).

6. Method according to claim 5, wherein the provision of the second measured phase distribution is a measurement of a phase distribution of an MR image after a second evolution period different from the first evolution period.

7. Method according to one of claims 1 to 4, wherein the provision of at least one second phase value of the region of interest (21) is a definition of a global phase value.

8. MR system for the determination of a phase distribution of a region of interest (21), wherein the MR system comprises an MR control unit (13) and a storage unit (16), wherein the storage unit (16) stores control information that can be executed by the MR control unit (13), and wherein the MR system is embodied to carry out the following steps when the control information is executed in the MR control unit (13):

   - provision of a first measured phase distribution of the region of interest (21) which is based at least on the known magnetic field distribution in the region of interest;
   - provision of at least one second phase value of the region of interest (21);
   - determination of a phase-difference distribution between the first measured phase distribution and the at least one second measured phase value in order to generate a combination-phase distribution, wherein the phase values of the combination-phase distribution are restricted to the presentation interval [-π;+π];
   - calculation of a correction-phase distribution from a divergent magnetic field distribution known a priori in absolute values in the region of interest (21), which is substantially not induced by an object of interest, wherein the phase values of the correction-phase distribution are not restricted to the presentation interval [-π;+π] (S44);
   - generation of a corrected combination-phase distribution using the correction-phase distribution and the combination-phase distribution, wherein the phase values of the corrected combination-phase distribution are restricted to the presentation interval [-π;+π]; and
   - generation of an absolute combination-phase distribution from the corrected combination-phase distribution using a phase-unwrapping technique, wherein the phase values of the absolute combination-phase distribution are not restricted to the presentation interval [-π;+π],

   **characterised in that** the known magnetic field distribution in the region of interest (21) is known a priori in absolute values and is substantially not induced by an object of interest.

9. MR system for the determination of a phase distribution of a region of interest (21), wherein the MR system comprises an MR control unit (13) and a storage unit (16), wherein the storage unit (16) stores control information that can be executed by the MR control unit (13), and wherein the MR system is embodied to carry out the method of one of claims 2 to 7 when the control information is executed in the MR control unit (13).

10. Computer program product comprising a program, which can be loaded directly into a memory of an MR control unit (13) of an MR system, with program means for carrying out the steps of the method according to one of claims 1 to 7 when the program is executed in the MR control unit (13) of the MR system.

11. Electronically readable data carrier with electronically readable control information stored thereupon, which is embodied to carry out the method according to one of claims 1 to 7 when the data carrier is used in an MR control unit (13) of an MR system.

**Revendications**

1. Procédé de détermination d'une répartition de phases d'une partie à examiner, répartition qui est déterminée pour la production d'une image RM de la partie (21) à examiner au moyen d'une installation RM, dans lequel on tient compte d'une répartition connue du champ magnétique dans la partie (21) à examiner, comprenant les stades suivants :

   - on se procure une première répartition de phases mesurée, qui repose (21, S41) au moins sur la répartition du champ magnétique connue dans la partie à examiner ;
   - on se procure au moins une deuxième valeur de phases de la partie (21, S42) à examiner ;
   - on détermine une répartition de différence de phases entre la première répartition de phases mesurée et la au moins une deuxième valeur de phases pour produire une répartition de phases de combinaison, les valeurs de phases de la répartition de phases de combinaison étant limitées à l'intervalle $[-\pi\ ;\ +\pi]$ de représentation (S43) ;
   - on calcule une répartition de phases de correction à partir de la répartition de champ magnétique connue dans la partie (21) à examiner, les valeurs de phases de la répartition de phases de correction n'étant pas limitées à l'intervalle $[-\pi\ ;\ +\pi]$ de représentation (S44) ;
   - on produit une répartition de phases de combinaison de correction, en utilisant la répartition de phases de correction et la répartition de phases de combinaison, les valeurs de phases de la répartition de phases de combinaison de correction étant limitées à l'intervalle $[-\pi\ ;\ +\pi]$ de représentation (S45) ; et
   - on produit une répartition de phases de combinaison absolue à partir de la répartition de phases de combinaison de correction par une technique de phase-unwrapping, les valeurs de phases de la répartition de phases de combinaison absolue n'étant pas limitées à l'intervalle $[-\pi\ ;\ +\pi]$ de représentation (S46) ;

   **caractérisé en ce que** la répartition du champ magnétique connue dans la partie (21) à examiner est connue, à priori, en valeurs absolues et n'est sensiblement pas induite par un objet à examiner.

2. Procédé suivant la revendication 1, dans lequel le procédé comprend, en outre, le stade suivant :

   - production d'une répartition de phases de combinaison absolue de correction, qui n'est pas limitée à l'intervalle $[-\pi\ ;\ +\pi]$ de représentation, par addition de la répartition de phases de correction à la répartition de phases de combinaison absolue pour la détermination d'une répartition de champ B0.

3. Procédé suivant la revendication 1 ou 2, dans lequel on détermine la répartition $\varphi_{korr}$ (r) de phases de correction par la formule

$$\varphi_{korr}(\ r\ )\ =\ \gamma \Delta B_0(\ r\ ) T_{eff}$$

   dans laquelle $\gamma$ est le rapport gyromagnétique, $\Delta B_0(r)$ est une répartition du champ magnétique dans la partie (21) à examiner, qui n'est pas induite par l'objet à examiner, r est le vecteur local dans la partie (21) à examiner et $T_{eff}$ est un temps d'évolution effectif.

4. Procédé suivant l'une des revendications précédentes, dans lequel la détermination de la première répartition de phases mesurée est une mesure d'une répartition de phases d'une image RM de la partie (21) à examiner après une première durée d'évolution.

5. Procédé suivant l'une des revendications 1 à 4, dans lequel la mise à disposition d'au moins une deuxième valeur de phase est une mise à disposition d'une deuxième répartition de phases mesurée de la partie (21) à examiner.

6. Procédé suivant la revendication 5, dans lequel la mise à disposition de la deuxième répartition de phases mesurée est une mesure de répartition de phases d'une image RM après une deuxième durée d'évolution différente de la première durée d'évolution.

7. Procédé suivant l'une des revendications 1 à 4, dans lequel la mise à disposition d'au moins une deuxième valeur de phase de la partie (21) à examiner est une fixation d'une valeur de phase globale.

8. Installation RM de détermination d'une répartition de phases d'une partie (21) à examiner, l'installation RM ayant une unité (13) de commande RM et une unité (16) de mise en mémoire, l'unité (16) de mise en mémoire mettant en mémoire de l'information de commande pouvant être réalisée par l'unité (13) de commande RM, et dans lequel l'installation RM est constituée pour effectuer, lors de l'exécution des informations de commande dans l'unité (13) de commande RM, les stades suivants :

   - mise à disposition d'une première répartition de phases mesurée de la partie (21) à examiner, répartition qui repose au moins sur la répartition du champ magnétique connue dans la partie à examiner ;
   - mise à disposition d'au moins une deuxième valeur de phases de la partie (21) à examiner ;
   - détermination d'une répartition de différence de phases entre la première répartition de phases mesurée et la au moins une deuxième valeur de phases pour produire une répartition de phases de combinaison, les valeurs de phase de la répartition de phases de combinaison étant limitées à l'intervalle $[-\pi \; ; +\pi]$ de représentation ;
   - calcul d'une répartition de phases de correction à partir d'une répartition du champ magnétique d'écart connue, à priori, en valeurs absolues dans la partie (21) à examiner, qui n'est sensiblement pas induite par un objet à examiner, les valeurs de phase de la répartition de phases de correction n'étant pas limitées à l'intervalle $[-\pi \; ; +\pi]$ de représentation (S44) ;
   - production d'une répartition de phases de combinaison de correction en utilisant la répartition de phases de correction et la répartition de phases de combinaison, les valeurs de phase de la répartition de phases de combinaison de correction étant limitées à l'intervalle $[-\pi \; ; +\pi]$ de représentation ; et
   - production d'une répartition de phases de combinaison absolue à partir de la répartition de phases de combinaison de correction par une technique de phase-unwrapping, les valeurs de phase de la répartition de phases de combinaison absolue n'étant pas limitées à l'intervalle $[-\pi \; ; +\pi]$ de représentation ;

   **caractérisé en ce que** la répartition du champ magnétique connue dans la partie (21) à examiner est connue, à priori, en valeurs absolues et n'est sensiblement pas induite par un objet à examiner.

9. Installation RM de détermination d'une répartition de phases d'une partie (21) à examiner, l'installation RM ayant une unité (13) de commande RM et une unité (16) de mise en mémoire, l'unité (16) de mise en mémoire mettant en mémoire des informations de commande pouvant être réalisées par l'unité (13) de commande RM et l'installation RM étant constituée pour effectuer, lors de l'exécution des informations de commande dans l'unité (13) de commande RM, le procédé suivant l'une des revendications 2 à 7.

10. Produit de programme d'ordinateur, qui comprend un programme, qui peut être chargé directement dans une mémoire d'une unité (13) de commande RM d'une installation RM, comprenant des moyens de programme pour effectuer les stades du procédé suivant l'une des revendications 1 à 7, lorsque le programme est exécuté dans l'unité (13) de commande RM de l'installation RM.

11. Support de données déchiffrables électroniquement, comprenant des informations de commande pouvant être déchiffrées électroniquement, qui y sont minorisées et qui sont conformées de manière à effectuer, lors de l'utilisation du support de données dans une unité (13) de commande RM d'une installation RM, le procédé suivant l'une des revendications 1 à 7.

# FIG 1

## FIG 2a

## FIG 2b

## FIG 3a

## FIG 3b

## FIG 4a

## FIG 4b

## FIG 5a

## FIG 5b

## FIG 6a

## FIG 6b

FIG 7

FIG 8

# FIG 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0390175 A2

**In der Beschreibung aufgeführte Nicht-Patentliteratur**

- Computing Magnetic Susceptibility Maps from Gradient Recalled Echo MRI for use in Multiple Sclerosis Studies. **GRANT YANG.** Thesis. The Ohio State University, 2013

- **JOSEPH BORELLO et al.** Chemical shift-based true water and fat images: regional phase correction of modified spin-echo MR images. *Radiology,* 1987, vol. 164.2, 531-537